# FASCICULE DE BREVET EUROPEEN

(11) **EP 4 021 278 B1**
(45) Date de publication et mention de la délivrance du brevet: **08.01.2025**
(21) Numéro de dépôt: 20753383.7
(22) Date de dépôt: 13.08.2020
(51) Int. Cl.: A61B 5/00, A61N 2/00

(54) **CASQUE SUPPORT POUR DISPOSITIF DE MAGNETOENCEPHALOGRAPHIE**
STÜTZHELM FÜR EIN MAGNETOENZEPHALOGRAFIEGERÄT
SUPPORT HELMET FOR A MAGNETOENCEPHALOGRAPHY DEVICE

(30) Priorité: 30.08.2019 FR 1909583
(43) Date de publication de la demande: 06.07.2022
(73) Titulaire: Commissariat à l'Energie Atomique et aux Energies Alternatives, 75015 Paris (FR)
(72) Inventeur: LE PRADO, Matthieu, 38054 Grenoble Cedex 9 (FR); LABYT, Etienne, 38054 Grenoble Cedex 9 (FR)
(74) Mandataire: Cabinet Beaumont
(86) Numéro de dépôt international: PCT/EP2020/072807
(87) Numéro de publication internationale: WO 2021/037584

(56) Documents cités:
- EP-A1- 0 595 227
- US-A1- 2007 225 585
- US-A1- 2012 226 127
- US-A1- 2014 121 491
- US-A1- 2015 257 673
- US-A1- 2017 123 495

## Description

### Domaine technique

La présente description concerne de façon générale les dispositifs de magnétoencéphalographie, et vise plus particulièrement un casque support pour dispositif de magnétoencéphalographie, et un dispositif de magnétoencéphalographie comportant un tel casque.

Le document US2012/226127 décrit un exemple d'un dispositif pour positionner des électrodes sur la tête d'un utilisateur.

### Technique antérieure

Un dispositif de magnétoencéphalographie a pour objet d'acquérir une image des champs magnétiques générés par le cerveau.

Les dispositifs de magnétoencéphalographie existants utilisent des magnétomètres de type SQUID (de l'anglais « Superconducting QUantum Interference Device » - dispositif supraconducteur à interférence quantique). Ces magnétomètres sont baignés dans un fluide cryogénique. Il en résulte un encombrement important et une absence de modularité du dispositif. En particulier, dans les dispositifs de magnétoencéphalographie existants, les magnétomètres ont une position fixe par rapport à un casque support dans lequel l'utilisateur vient placer sa tête lors d'une phase d'acquisition d'une image. Ce casque est généralement prévu pour être adapté aux têtes les plus grosses. Dans la pratique, la tête de l'utilisateur peut ainsi se trouver à plusieurs centimètres des bords du casque, et donc des magnétomètres. Les champs mesurés par les magnétomètres s'en trouvent atténués, ce qui dégrade la qualité des images acquise.

On a récemment proposé, en alternative aux magnétomètres de type SQUID, d'utiliser des magnétomètres à pompage optique. Les magnétomètres à pompage optique n'ont en effet pas besoin d'être refroidis par un fluide cryogénique, ce qui permet de réaliser des dispositifs de magnétoencéphalographie moins encombrants et moins coûteux.

Il serait souhaitable d'améliorer au moins en partie certains aspects des dispositifs de magnétoencéphalographie à base de magnétomètres à pompage optique.

### Résumé de l'invention

Pour cela, un mode de réalisation prévoit un casque support pour dispositif d'imagerie ou de traitement médical, comportant une coiffe munie d'une pluralité d'ouvertures traversantes, chaque ouverture étant adaptée à recevoir un module élémentaire d'imagerie ou de traitement monté coulissant dans l'ouverture selon un axe sensiblement orthogonal à la coiffe.

Le casque comporte en outre un dispositif de serrage adapté à exercer sur chaque module une pression en direction de l'intérieur de la coiffe, de façon à maintenir le module en appui contre la tête d'un utilisateur.

Le dispositif de serrage comprend un lacet adapté à coulisser librement dans un passage prévu à cet effet sur chaque module.

Selon un mode de réalisation, le dispositif de serrage comprend en outre, fixé sur la coiffe, au moins un élément de serrage autobloquant du lacet.

Selon un mode de réalisation, la coiffe est en un matériau rigide.

Selon un mode de réalisation, la coiffe comprend, au niveau d'un bord de chaque ouverture, un ergot de guidage du module élémentaire destiné à coopérer avec une rainure de guidage correspondante du module élémentaire.

Un autre mode de réalisation prévoit un dispositif d'imagerie ou de traitement médical, comportant un casque support tel que défini ci-dessus, et une pluralité de modules élémentaires d'imagerie ou de traitement montés respectivement dans les ouvertures de la coiffe du casque.

Selon un mode de réalisation, les modules élémentaires sont des magnétomètres à pompage optique.

L'invention est définie dans les revendications attenantes.

### Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :
la figure 1 représente de façon schématique et partielle un exemple d'un dispositif de magnétoencéphalographie à base de magnétomètres à pompage optique selon un mode de réalisation ;
la figure 2 est une vue en coupe plus détaillée d'une portion du dispositif de la figure 1 ;
la figure 3 est une vue de dessous d'une portion d'un casque support du dispositif de la figure 1 ;
la figure 4 est une vue latérale d'un magnétomètre élémentaire du dispositif de la figure 1 ;
la figure 5 est une vue de dessous d'un magnétomètre élémentaire du dispositif de la figure 1 ; et
la figure 6 représente de façon schématique et partielle une variante de réalisation du dispositif de magnétoencéphalographie de la figure 1.

### Description des modes de réalisation

De mêmes éléments ont été désignés par de mêmes références dans les différentes figures. En particulier, les éléments structurels et/ou fonctionnels communs aux différents modes de réalisation peuvent présenter les mêmes références et peuvent disposer de propriétés structurelles, dimensionnelles et matérielles identiques.

Par souci de clarté, seuls les étapes et éléments utiles à la compréhension des modes de réalisation décrits ont été représentés et sont détaillés. En particulier, la structure interne des magnétomètres à pompage optique des dispositifs décrits n'a pas été détaillée, les modes de réalisation décrits étant compatibles avec la plupart des structures connues de magnétomètres à pompage optique. De plus, les circuits périphériques de contrôle et de traitement reliés aux magnétomètres à pompage optique des dispositifs décrits n'ont pas été détaillés, les modes de réalisation décrits étant compatibles avec les circuits de contrôle et de traitement usuellement prévus dans les dispositifs de magnétoencéphalographie à base de magnétomètres à pompage optique, ou la réalisation de ces circuits étant à la portée de la personne du métier.

Sauf précision contraire, lorsque l'on fait référence à deux éléments connectés entre eux, cela signifie directement connectés sans éléments intermédiaires autres que des conducteurs, et lorsque l'on fait référence à deux éléments reliés ou couplés entre eux, cela signifie que ces deux éléments peuvent être connectés ou être reliés ou couplés par l'intermédiaire d'un ou plusieurs autres éléments.

Dans la description qui suit, lorsque l'on fait référence à des qualificatifs de position absolue, tels que les termes "avant", "arrière", "haut", "bas", "gauche", "droite", etc., ou relative, tels que les termes "dessus", "dessous", "supérieur", "inférieur", etc., ou à des qualificatifs d'orientation, tels que les termes "horizontal", "vertical", etc., il est fait référence sauf précision contraire à l'orientation des figures ou à un dispositif dans une position normale d'utilisation.

Sauf précision contraire, les expressions "environ", "approximativement", "sensiblement", et "de l'ordre de" signifient à 10 % près, de préférence à 5 % près.

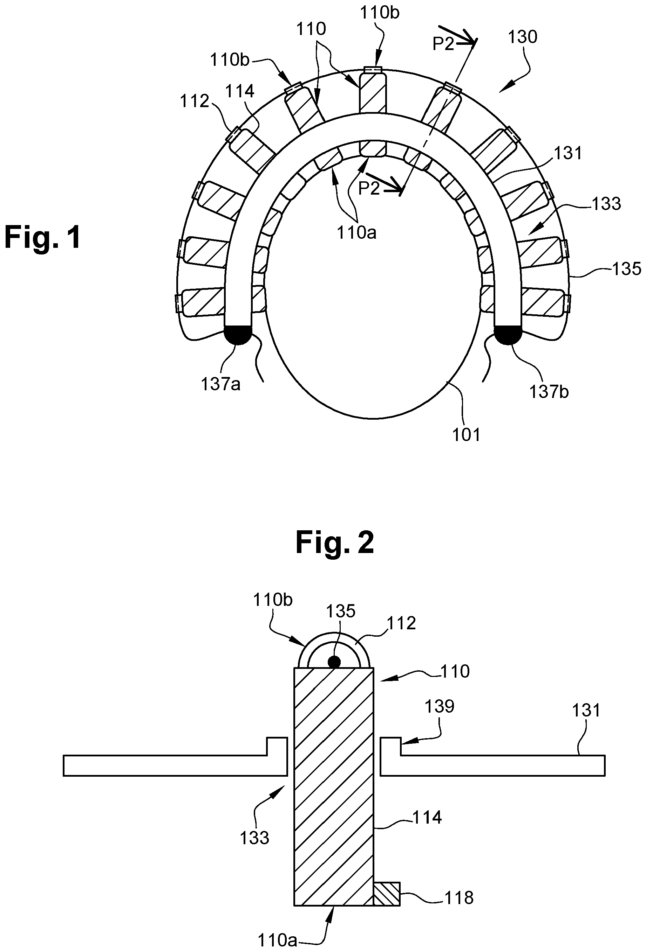

La figure 1 représente de façon schématique et partielle un exemple d'un dispositif de magnétoencéphalographie à base de magnétomètres à pompage optique selon un mode de réalisation.

Le dispositif de la figure 1 comprend un casque support 130, sur lequel sont fixés une pluralité de magnétomètres à pompage optique élémentaires 110, par exemple identiques ou similaires aux dispersions de fabrication près. A titre d'exemple, le dispositif comporte 20 à 200 magnétomètres élémentaires 110. Les magnétomètres 110 sont par exemple régulièrement répartis, à pas constant, sur la surface du casque.

Le casque support 130 du dispositif de la figure 1 comprend une coiffe 131 destinée à être placée sur la tête 101 d'un utilisateur. La coiffe 131 peut avoir une forme bombée, par exemple une forme de portion de sphère ou d'ellipsoïde. La coiffe 131 est munie d'une pluralité d'ouvertures traversantes 133, par exemple sensiblement de mêmes dimensions, chaque ouverture étant adaptée à recevoir un magnétomètre élémentaire 110. La coiffe 131 peut être en un matériau rigide, par exemple en plastique.

Chaque magnétomètre élémentaire 110, une fois placé dans une ouverture élémentaire 133 de la coiffe 131, a une première face 110a située à l'intérieur de la coiffe, tournée vers la tête de l'utilisateur, et une deuxième face 110b opposée à la face 110a, située à l'extérieur de la coiffe.

Chaque magnétomètre 110 est adapté à coulisser dans l'ouverture correspondante 133 de la coiffe 131, selon un axe sensiblement orthogonal à la coiffe. Plus particulièrement, dans cet exemple, chaque magnétomètre peut se déplacer dans l'ouverture 133 selon un unique degré de liberté en translation selon un axe sensiblement orthogonal à la coiffe. Par axe sensiblement orthogonal à la coiffe, on entend ici un axe formant un angle inférieur à 30 degrés, et de préférence inférieur à 20 degrés, en valeur absolue, avec l'axe normal à la surface extérieure de la coiffe au niveau du centre de l'ouverture 133.

A titre d'exemple, les magnétomètres 110 ont, en section transversale, c'est-à-dire selon un plan sensiblement orthogonal à leur axe de coulissement, une forme sensiblement identique à celle des ouvertures 133. Les dimensions des ouvertures 133 peuvent être très légèrement supérieures à celles des magnétomètres de façon à permettre le passage des magnétomètres dans les ouvertures. Ceci permet d'autoriser un déplacement en translation des magnétomètres 110 selon leur axe longitudinal, tout en bloquant les translations dans les autres directions. A titre d'exemple, les dimensions des ouvertures 133 sont supérieures de 1 à 5% aux dimensions transversales des magnétomètres 110. A titre d'exemple, en vue de dessus, les dimensions des ouvertures 133, correspondant sensiblement aux dimensions transversales des magnétomètres 110, sont comprises entre 10 et 40 millimètres, par exemple de l'ordre de 20 millimètres.

Les magnétomètres 110 ont par exemple une forme générale parallélépipédique, par exemple parallélépipédique rectangle. Plus généralement, les magnétomètres 110 peuvent avoir toute autre forme, par exemple une forme cylindrique.

Dans cet exemple, les magnétomètres 110 peuvent se déplacer indépendamment les uns des autres le long de leurs axes de coulissement respectifs. Autrement dit, les magnétomètres 110 ne sont pas solidaires mécaniquement les uns des autres.

Le casque support 130 de la figure 1 comprend en outre un dispositif de serrage adapté à appliquer sur chaque magnétomètre 110 une pression en direction de l'intérieur de la coiffe, parallèlement à son axe de coulissement, de façon à maintenir la face 110a du magnétomètre en appui contre la tête de l'utilisateur, par exemple en contact avec la tête de l'utilisateur.

Dans l'exemple de la figure 1, le dispositif de serrage comprend un lacet 135 adapté à coulisser librement dans un passage ou guide prévu à cet effet sur chaque magnétomètre 110. Dans l'exemple représenté, chaque magnétomètre 110 comprend, du côté de sa face 110b opposée à la tête de l'utilisateur, une goulotte de passage 112 fixée sur un boîtier de protection 114 du magnétomètre, le lacet 135 passant dans les goulottes respectives des différents magnétomètres. Le lacet 135 est par exemple élastique.

Dans l'exemple de la figure 1, le dispositif de serrage comprend en outre deux éléments de serrage autobloquants 137a et 137b du lacet 135. Dans cet exemple, les éléments de serrage autobloquants 137a et 137b sont fixés sur un bord de la coiffe 131, respectivement à gauche et à droite du visage de l'utilisateur. Le lacet 135 a une première extrémité maintenue par l'élément de serrage 137a et une deuxième extrémité maintenue par l'élément de serrage 137b. Le serrage du lacet 135 au moyen des éléments 137a et 137b permet d'appliquer aux différents magnétomètres 110 une pression apte à maintenir la face 110a de chaque magnétomètre en appui contre le crâne de l'utilisateur, par exemple en contact avec la tête de l'utilisateur.

En pratique, les magnétomètres élémentaires peuvent être disposés selon plusieurs rangées de plusieurs magnétomètres chacune. Le dispositif de serrage peut comporter un lacet 135 par rangée de magnétomètres. Les différents lacets 135 passent par exemple dans les mêmes deux éléments de serrage autobloquants 137a et 137b. A titre de variante, le dispositif de serrage comprend un unique élément de serrage autobloquant fixé à la coiffe 131, chaque lacet 135 ayant une première extrémité directement fixée à la coiffe et une deuxième extrémité maintenue par l'élément de serrage autobloquant. A titre de variante, différents lacets sont maintenus par des éléments de serrage autobloquants distincts. Les lacets sont par exemple répartis en plusieurs groupes d'un ou plusieurs lacets par groupe, le dispositif de serrage comportant, fixés à la coiffe, un ou deux éléments de serrage autobloquants par groupe de lacets.

La figure 2 est une vue en coupe plus détaillée d'une portion du dispositif de la figure 1, selon le plan P2 de la figure 1. Sur la figure 2, un unique magnétomètre 110 est représenté, ainsi qu'une portion correspondante de la coiffe 131.

Comme cela apparaît sur la figure 2, dans cet exemple, la coiffe 131 comprend, en bordure de chaque ouverture 133, un épaulement 139 (non visible sur la figure 1) permettant notamment de faciliter le guidage du magnétomètre 110. A titre d'exemple, l'épaulement 139 forme, à la périphérie de l'ouverture 133, une surépaisseur en saillie de la face extérieure de la coiffe 131. A titre d'exemple, en vue de dessus, l'épaulement 139 entoure entièrement l'ouverture 133. Dans l'exemple représenté, le bord intérieur de l'épaulement 139 est superposé au bord de l'ouverture 133. A titre d'exemple, l'épaulement 139 présente une épaisseur de 5 à 25 millimètres, par exemple de l'ordre de 10 millimètres, en sus de l'épaisseur de la coiffe 131.

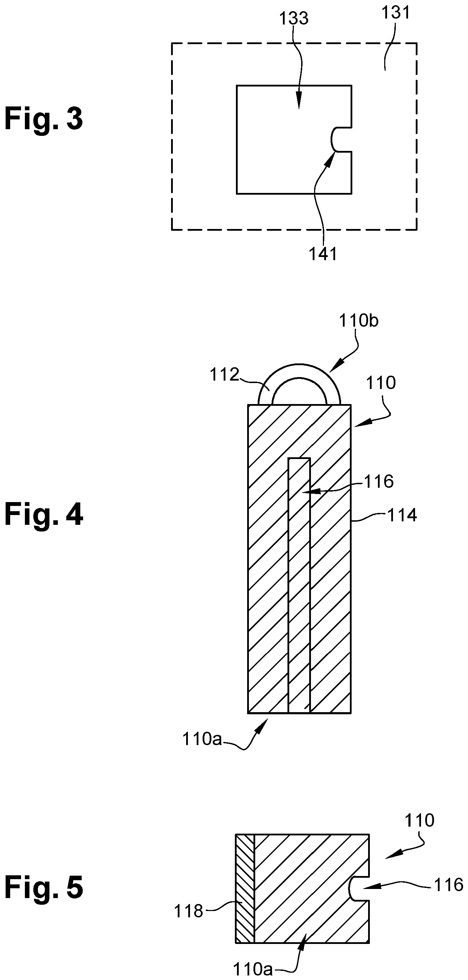

La figure 3 est une vue de dessous agrandie d'une portion de la coiffe 131 du dispositif de la figure 1. Sur la figure 3, une unique ouverture 133 a été représentée.

La figure 4 représente quant à elle une vue latérale agrandie d'un magnétomètre élémentaire 110 du dispositif de la figure 1.

La figure 5 représente une vue de dessous du magnétomètre élémentaire 110 de la figure 4.

Comme cela apparaît sur la figure 3, la coiffe 131 peut comporter, à chaque ouverture 133 un ergot de guidage 141 en saillie d'un bord de l'ouverture. Chaque magnétomètre élémentaire 110 peut quant à lui comporter, sur une face latérale, une rainure ou gorge de guidage 116 (figures 4 et 5), s'étendant sur tout ou partie de la longueur du magnétomètre, dans la direction de coulissement du magnétomètre. L'ergot 141 est destiné à coopérer avec la rainure 116 pour assurer le guidage du magnétomètre dans l'ouverture 133. L'ergot 141 et la rainure 116 servent en outre de détrompeur pour fixer l'orientation du magnétomètre 110 par rapport à la coiffe. A titre d'exemple, la rainure 116 s'étend depuis la face inférieure 110a du magnétomètre, sur une partie seulement de la hauteur du magnétomètre, par exemple sur 50 à 90% de la hauteur du magnétomètre. L'interruption de la rainure 116 avant la face supérieure 110b du magnétomètre permet de former une butée évitant que le magnétomètre ne tombe vers l'intérieur de la coiffe 131, par exemple lorsque la tête de l'utilisateur n'est pas présente dans le casque. Le prolongement de la rainure 116 jusqu'à la face inférieure 110a du magnétomètre permet l'insertion du magnétomètre dans l'ouverture 133, depuis la face supérieure de la coiffe 131.

Comme cela apparaît sur les figures 2 et 5, chaque magnétomètre élémentaire 110 peut de plus comprendre, sur une de ses faces latérales, par exemple sa face opposée à la rainure 116, au voisinage de sa face inférieure 110a, un épaulement 118 fixé de façon amovible au boîtier 114 du magnétomètre, par exemple au moyen d'une vis ou d'un système de clipsage. L'épaulement 118 sert de butée permettant d'éviter que le magnétomètre ne sorte du côté de la face extérieure de la coiffe 131, par exemple si le casque est retourné. L'épaulement 118 est fixé de manière amovible pour permettre son retrait lors de l'insertion du magnétomètre 110 dans l'ouverture 133 ou lors du retrait du magnétomètre 110 de l'ouverture 133.

On notera que les modes de réalisation décrits ne se limitent pas aux exemples particuliers de mécanismes de guidage et de butée décrits ci-dessus. Plus généralement, tous autres mécanismes adaptés à assurer les fonctions décrites ci-dessus pourront être utilisé. Par exemple, l'ergot fixe 141 peut être remplacé par un ergot rétractable à ressort. De façon similaire, l'épaulement 118 peut être remplacé par un ergot rétractable à ressort.

Bien que non représenté, le dispositif de magnétoencéphalographie de la figure 1 peut en outre comporter un circuit central de contrôle et de traitement relié aux magnétomètres à pompage optique 110, par exemple par liaison filaire ou par liaison radio.

Un avantage du dispositif de magnétoencéphalographie de la figure 1 est qu'il permet d'appliquer les magnétomètres au plus près de la tête de l'utilisateur, quelle que soit la taille de cette dernière.

De plus, dans la mesure où les magnétomètres sont fixés de manière amovible au casque de support, et du fait du prix de revient relativement faible du casque support, plusieurs tailles de casques différentes peuvent être prévues, par exemple 3 ou 4 tailles de casques, pour s'adapter à différentes gammes de dimensions de tête (par exemple une taille bébé, une taille enfant, et une taille adulte).

Par ailleurs, les magnétomètres étant fixés de manière amovible au casque support, le nombre de magnétomètres et leur position sur le casque peuvent être adaptés en fonction de la mesure que l'on souhaite effectuer. Ainsi, lors d'une mesure, certaines ouvertures 133 de la coiffe 131 peuvent rester libres, c'est-à-dire non équipées d'un magnétomètre.

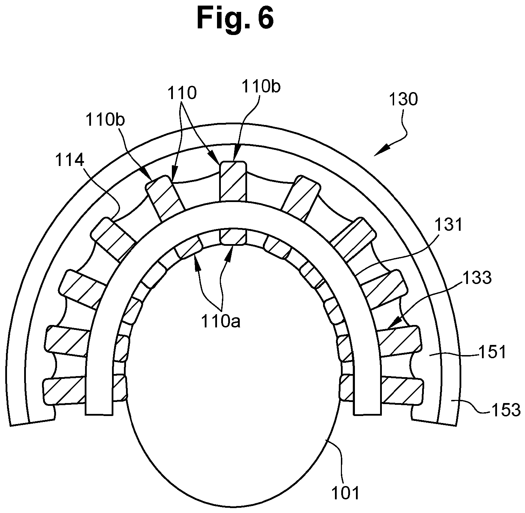

La figure 6 représente de façon schématique et partielle une variante de réalisation du dispositif de magnétoencéphalographie de la figure 1.

Le dispositif de la figure 6 diffère du dispositif de la figure 1 principalement par la nature du dispositif de serrage utilisé pour appliquer, sur chaque magnétomètre élémentaire 110, une pression en direction de l'intérieur de la coiffe, permettant de maintenir la face 110a du magnétomètre en appui contre la tête de l'utilisateur.

Dans l'exemple de la figure 6, le dispositif de serrage comprend un coussin gonflable 151, par exemple un coussin d'air, situé à l'extérieur de la coiffe 131. Le dispositif de serrage de la figure 6 comprend en outre une coque extérieure 153 située à l'extérieur de la coiffe 131, du côté du coussin gonflable 151 opposé à la coiffe 131. La coque 153 est par exemple une coque rigide solidaire mécaniquement de la coiffe 131. Le coussin gonflable 151 présente une face intérieure en appui contre les faces extérieures 110b des magnétomètres élémentaires 110, et une face extérieure en appui contre une face intérieure de la coque extérieure 153. Une pompe, non représentée, peut être prévue pour ajuster la pression du coussin d'air 151 de façon à maintenir les magnétomètres 110 en appui contre la tête de l'utilisateur. De préférence, la pompe sera alors placée à l'extérieur d'un blindage magnétique (non représenté) prévu pour isoler les magnétomètres 110 d'éventuels champs magnétiques parasites extérieurs.

Plus généralement, d'autres dispositifs de serrage pourront être prévus pour assurer le maintien des magnétomètres en appui contre la tête de l'utilisateur, par exemple des dispositifs à ressort.

Divers modes de réalisation et variantes ont été décrits. La personne du métier comprendra que certaines caractéristiques de ces divers modes de réalisation et variantes pourraient être combinées, et d'autres variantes apparaîtront à la personne du métier. En particulier, les modes de réalisation décrits ne se limitent pas aux exemples particuliers de dimensions mentionnés dans la présente description.

Par ailleurs, bien que l'on ait décrit ci-dessus des dispositifs pour des applications de magnétoencéphalographie, ces dispositifs pourront être adaptés à d'autres applications d'imagerie médicale ou de traitement médical du cerveau ou de la tête, en remplaçant les magnétomètres à pompage optique 110 par d'autres modules élémentaires d'imagerie ou de traitement. A titre d'exemple, les magnétomètres à pompage optique 110 peuvent être remplacés par d'autres types de capteurs, par exemple optiques, térahertz, acoustiques, etc., ou encore par des modules élémentaires de traitement ou de stimulation, par exemple par ondes radio ou par champ magnétique.

## Revendications

1. Casque support (130) pour dispositif d'imagerie ou de traitement médical, comportant une coiffe (131) munie d'une pluralité **d'ouvertures** traversantes (133), chaque ouverture étant adaptée à recevoir un module élémentaire (110) **d'imagerie** ou de traitement monté coulissant dans l'ouverture selon un axe sensiblement orthogonal à la coiffe, le casque comportant en outre un dispositif de serrage adapté à exercer sur chaque module (110) une pression en direction de **l'intérieur** de la coiffe (131), de façon à maintenir le module en appui contre la tête (101) d'un utilisateur, dans lequel le dispositif de serrage comprend un lacet (135) adapté à coulisser librement dans un passage (112) prévu à cet effet sur chaque module (110).

2. Casque (130) selon la revendication 1, dans lequel le dispositif de serrage comprend en outre, fixé sur la coiffe (131), au moins un élément de serrage autobloquant (137a, 137b) du lacet.

3. Casque (130) selon la revendication 1, dans lequel la coiffe (131) est en un matériau rigide.

4. Casque (130) selon l'une quelconque des revendications 1 à 3, dans lequel la coiffe (131) comprend, au niveau d'un bord de chaque ouverture (133), un ergot (141) de guidage du module élémentaire (110) destiné à coopérer avec une rainure de guidage (116) correspondante du module élémentaire (110).

5. Dispositif d'imagerie ou de traitement médical, comportant un casque support (130) selon l'une quelconque des revendications 1 à 4, et une pluralité de modules élémentaires (110) d'imagerie ou de traitement montés respectivement dans les ouvertures (133) de la coiffe (131) du casque.

6. Dispositif d'imagerie ou de traitement selon la revendication 5, dans lequel les modules élémentaires (110) sont des magnétomètres à pompage optique.

## Patentansprüche

1. Stütz- oder Trägerhelm (130) für eine medizinisches Bildgebungs- oder Behandlungsvorrichtung, die eine Kopfkappe (131) aufweist, die mit einer Vielzahl von Durchgangsöffnungen (133) versehen ist, wobei jede Öffnung zur Aufnahme eines elementaren Bildgebungs- oder Behandlungsmoduls (110) ausgelegt ist, das in der Öffnung so montiert ist, dass es entlang einer Achse gleitet, die im Wesentlichen orthogonal zu der Kopfkappe ist, wobei der Helm ferner eine Spannvorrichtung aufweist, die so ausgelegt ist, dass sie auf jedes Modul (110) einen Druck in Richtung des Inneren der Kopfkappe (131) ausübt, und zwar um das Modul auf tragende Weise gegen den Kopf (101) eines Benutzers zu drücken, wobei die Spannvorrichtung eine Schnur (135) aufweist, die so ausgelegt ist, dass sie frei in einem zu diesem Zweck an jedem Modul (110) vorgesehenen Durchgang (112) gleitet.

2. Helm (130) nach Anspruch 1, wobei die Spannvorrichtung außerdem mindestens ein selbstsicherndes Element (137a, 137b) zum Spannen der Schnur aufweist, die an der Kopfkappe (131) befestigt ist.

3. Helm (130) nach Anspruch 1, wobei die Kopfkappe (131) aus einem steifen Material besteht.

4. Helm (130) nach einem der Ansprüche 1 bis 3, wobei die Kopfkappe (131) auf Höhe eines Randes jeder Öffnung (133) einen Stift (141) zur Führung des Elementarmoduls (110) aufweist, der dazu bestimmt ist, mit einer entsprechenden Führungsnut (116) des Elementarmoduls (110) zusammenzuwirken.

5. Medizinische Bildgebungs- oder Behandlungsvorrichtung, die einen Stütz- oder Trägerhelm (130) gemäß einem der Ansprüche 1 bis 4 und eine Vielzahl von elementaren Bildgebungs- oder Behandlungsmodulen (110) aufweist, die jeweils in den Öffnungen (133) der Kopfkappe (131) des Helms montiert sind.

6. Bildgebungs- oder Behandlungsvorrichtung gemäß Anspruch 5, wobei die elementaren Module (110) optische Pumpmagnetometer sind.

## Claims

1. Support helmet (130) for a medical imaging or treatment device, comprising a head cap (131) provided with a plurality of through openings (133), each opening being adapted to receiving an elementary imaging or treatment module (110) assembled in the opening so as to slide along an axis substantially orthogonal to the head cap, the helmet further comprising a tightening device adapted to exerting on each module (110) a pressure towards the inside of the head cap (131), to hold the module bearing against a user's head (101), wherein the tightening device comprises a lace (135) adapted to freely sliding in a passage (112) provided for this purpose on each module (110).

2. Helmet (130) according to claim 1, wherein the tightening device further comprises, fastened on the head cap (131), at least one self-locking element (137a, 137b) for tightening the lace.

3. Helmet (130) according to claim 1, wherein the head cap (131) is made of a rigid material.

4. Helmet (130) according to any of claims 1 to 3, wherein the head cap (131) comprises, at the level of an edge of each opening (133), a pin (141) for guiding the elementary module (110), intended to cooperate with a corresponding guiding groove (116) of the elementary module (110).

5. Medical imaging or treatment device, comprising a support helmet (130) according to any of claims 1 to 4, and a plurality of elementary imaging or treatment modules (110) respectively assembled in the openings (133) of the head cap (131) of the helmet.

6. Imaging or treatment device according to claim 5, wherein the elementary modules (110) are optical pumping magnetometers.
